# EUROPEAN PATENT APPLICATION

(11) **EP 3 167 898 A1**
(43) Date of publication of application: **17.05.2017**
(21) Application number: 15194078.0
(22) Date of filing: 11.11.2015
(51) Int. Cl.: A61K 39/00

(54) **METHOD FOR OBTAINING TUMOR PEPTIDES AND USES THEREOF**

(71) Applicant: IEO - Istituto Europeo di Oncologia Srl, 20121 Milano (IT)
(72) Inventor: Rescigno, Maria, 20139 Milano (IT); Penna, Giuseppe, 20139 Milano (IT)
(74) Representative: Capasso, Olga

(57) **Abstract**

The present invention refers to a method for obtaining a cell culture supernatant or a fraction thereof comprising a specific tumor antigen peptide repertoire comprising the steps of: a) exposing in suitable conditions a tumor cell culture expressing said specific tumor antigen peptide repertoire to at least one Pattern Recognition Receptor (PRR) agonist and/or to one inflammatory cytokine to increase the opening of connexin-hemichannels (CxH); b) collecting the cell culture supernatant; and c) optionally obtaining a fraction of said supernatant, wherein said supernatant comprises MHC class I and MHC class II peptides and non-classical MHC molecules (HLA-E).

SEQUENCE LISTING

<110>  IEO - Istituto Europeo di Oncologia S.r.l.

<120>  Method for obtaining tumor peptides and uses thereof

<130>  BE 127168

<160>  1

<170>  PatentIn version 3.5

<210>  1
<211>  8
<212>  PRT
<213>  Artificial Sequence

<220>
<223>  H-2Kb restricted OVA octapeptide

<400>  1

Ser Ile Ile Asn Phe Glu Lys Leu
1               5

## Description

### ABSTRACT

The invention relates to a novel method of cancer vaccine generation. It is shown a methodology to open up connexin-hemichannels (CxH) in cancer cells. These channels can be successfully exploited to release tumor antigenic peptides directly in the extracellular milieu. These tumor peptides are able to induce an antitumor immune response in vivo. These peptides include both B and T cell epitopes and if injected in vivo they foster both antibody and cellular responses to tumor cells.

### BACKGROUND

Peptide vaccines are preparations made from synthetic epitopes that represent the minimal immunogenic region of a protein. This therapy is mainly applied to melanoma since it is one of the most immunogenic cancers.

The major advantages of peptide vaccines are that they are simple, safe, stable and economical, as well as easy to produce and store. They are easily characterized and analyzed by well-established techniques (Mocellin 2012). Also, peptides can be relatively easily modified in order to improve their immunogenicity, stability and solubility by the introduction of lipid, carbohydrate and phosphate groups. The major weakness of peptide-based vaccines is their inconsistent ability to stimulate effector T cells: although they are quite able to stimulate TA-specific CD4+ T cells, they are only poorly able to stimulate TA-specific CD8+ T cells. The use of naturally occurring peptides alone for anticancer vaccination is rarely followed by a tumor response, so various approaches were studying to overcome this limitation (Adams, Lowes et al. 2008). One approach can be the use of immunological adjuvants: the slow release of antigens have been recognized as a critical method in the induction of effective immune responses. Among the adjuvants in current use with cancer vaccines are aluminium salt, oil in water emulsion and nontoxic derivates from Salmonella and the saponins. A major new advance in this field has been the introduction of toll-like receptor ligands (TLRL) which potently activate APCs *in vivo.* There are various toll-like receptor agonists currently in use such as TLR7/8L (Imiquimod, Resiquimod) and TLR9L (CpG). Notably, some TLRLs such as TLR3L have pleiotropic effects, activating APCs as well as NK, and mediating tumor cell death (Fourcade, Sun et al. 2010). CpG is a potent adjuvant for peptide cancer vaccines, stimulating ex vivo detectable TA-specific CD8+ T cells in patients with advanced cancer (Speiser, Baumgaertner et al. 2008).

Another approach to improve the immunogenicity of peptides is to alter amino acids at HLA binding residues (called "anchor residues") to enhance their HLA binding affinity. These modifications can dramatically influence the conformation of the peptide/HLA groove inducing stronger anti-tumor immunity in mice and enhances the efficacy of T cell-induction in humans (Meijer, Dols et al. 2007). However, several findings suggest caution in the use of modified TAA (Tumor Associated Antigen) epitopes because in some cases they induce response *in vivo* that reduce the ability of immune system to control tumor growth.

A further approach is based on use of mimotopes, molecules that mimic an antigenic determinant of the nominal antigen and are capable of inducing antibody and cellular immune responses to the nominal antigen (Knittelfelder, Riemer et al. 2009) The rational of this strategy depends on the fact that mimotopes provide an alternative to natural T-cell epitopes for anticancer vaccination because they can recruit and stimulate T cell-repertoires that deviate from the repertoires engaged at the tumor site. Besides demonstrating therapeutic efficacy in preclinical models including melanoma, this approach has entered the clinical phase of experimentation (van Stipdonk, Badia- Martinez et al. 2009).

Additionally, a strategy widely applied for melanoma therapy is multi-epitope vaccine, which is believed to circumvent some limits of single epitope regimens. From the antimelanoma efficacy viewpoint, multiepitope vaccines have been shown to elicit adequate immune responses ex vivo (Parmiani, Castelli et al. 2002). However, modest or not significant therapeutic benefit has been so far reported in patients with advanced melanoma. Therefore, more work is needed in order to identify the correct formulation that might lead to therapeutic efficacy of peptide vaccine strategy. As a mechanism of immune evasion, tumor cells often have impaired ability to process and present tumor antigenic peptides (Chen, H.L. et al. Nat. Genet. 13, 210-213 (1996). Evans, M. et al. J. Immunol. 167, 5420- 5428 (2001). Alimonti, J. et al. Nat. Biotechnol. 18, 515-520 (2000)). Several steps in tumor antigen processing can be affected from their processing to their transport into the endoplasmic reticulum by TAP. This allows tumor cells to evade recognition by tumor-specific T cells. However, in these circumastances a new class of peptides called T-cell epitopes associated with impaired peptide processing, TEIPP, can be presented on the cell surface as there is lack of competition for presentation on MHC molecules (both classical and non-classical) by TAP-transported peptides. TEIPP have been proposed as a possible tool to induce an immune response to tumors (Van Hall T et al. NATURE MEDICINE VOLUME 12 [ NUMBER 4 [ APRIL 2006). However, there were little or no tools to study the identity of these peptides.

Dillman R. et al (Cancer Biotherapy & Radiopharmaceuticals, 2009, 24, p. 311) relates to an autologous tumor cell vaccines consisting of dendritic cells (DCS), derived from patient's peripheral blood cells cultured in (IL)-4 and granulocyte macrophage colony-stimulating factor (GM-CSF), which had phagocytosed irradiated autologous tumor cells from a continuously proliferating, self-renewing, autologous tumor cell (TC) culture.

WO2009/040413 relates to a process to obtain activated antigen-presenting cells that are useful for therapies against cancer and immune system-related diseases, by means of a cellular composition that contributes to stimulate the activated antigen-presenting cells to induce specific immune response against tumours. The method induces the differentiation of monocytes in APC (dendritic cells) by stimulation in culture using cytokines, growth factors and/or mixture of lysate or extracts of tumour cells.

Mendoza-Naranjo A, et al, and Salazar-Onfray, J. Immunol. 2007; 178;6949-6957 describes the use of melanoma cell lysate stimulated with TNFalfa to induce gap junctions to promote Ag transfer between ex vivo produced hDCs from melanoma patients.

WO202008019366 relates to methods and compositions for increased priming of T-cells through cross presentation of exogenous antigens. It refers to particles (S. Cerevisiae) on the surface of which the antigen is attached, and administering the antigen preparation to the animal, wherein the particles are taken up by antigen presenting cells (APC) of the animal via phagocytosis.

US 20090324651 relates to methods for stimulating an immune response using bacterial antigen delivery system. It relates to the use of the type III secretion system of bacteria to stimulate immune responses against tumor antigen(s) for treating antigen-loss variant tumors. Methods are provided for stimulating and/or increasing an immune response against tumor antigens.

The prior art document also relates to the preparation of antigen presenting cells from peripheral blood mononuclear cells using bacteria having a type III secretion system. The method refers to the culture of PBMCs, previously contacted with an avirulent bacteria (such as S. typhimurium) expressing a tumor antigen, and isolating antigen presenting cells. Salmonella acts as vehicle of the tumoral antigen (previously "loaded" on bacteria) to the APC cell (degradation of antigen is still made by the APC).

Eugenin E. A. et al, and Juan C. Saez. J. Immunol 2003, 170:1320-1328 discloses that TNF-alfa plus IFN-gamma induce connexin 43 expression and formation of gap junctions between human monocytes and macrophages.

Elgueta R. et al, and Saez J. J Immunol 2009, 183(1):277-84 reports the formation of gap junctions between DCs and T cells and their role on T cell activation during Ag presentation by DCs. WO2004/050855 relates to a one-step method for producing antigen loaded dendritic cells vaccine comprising an activator such as TNF alpha preferably in combination with at least one growth factor such as GM-CSF and at least one soluble or particulate antigen.

AU2014271235 relates to peptides, nucleic acids and cells for use in immunotherapeutic methods. In particular, the invention relates to the immunotherapy of cancer. The invention furthermore relates to tumor associated cytotoxic T cell (CTL) peptide epitopes, alone or in combination with other tumor-associated peptides that serve as active pharmaceutical ingredients of vaccine compositions that stimulate anti-tumor immune responses. The invention relates to 30 peptide sequences and their variants derived from HLA class I and class II molecules of human tumor cells that can be used in vaccine compositions for eliciting anti-tumor immune responses. WO2007028573 relates to tumour-associated T-helper cell peptide epitopes, alone or in combination with other tumour-associated peptides, that serve as active pharmaceutical ingredients of vaccine compositions which stimulate anti-tumour immune responses. In particular, the application relates to two novel peptide sequences derived from HLA class II molecules of human tumour cell lines which can be used in vaccine compositions for eliciting anti-tumour immune responses.

WO98/15282 relates to an immunogenic composition comprising at least one protein from TLP (tumor-liberated particles, proteic complexes present in human tumor cells) or a fragment thereof, and in particular to the compositions wherein said fragments can comprise at least one of the peptides defined by particular sequences, suitable in therapy against tumoral diseases, and in particular against NSCLC and uro-genital cancer.

WO94/01458 relates to peptides comprised within the 100 KDa protein of the TLP complex (i.e., released proteins from tumors) having antigenic activity as well as antibodies thereof, able to react with TLP for diagnostic and clinical purposes.

WO2009102909 provides tumor-associated HLA-restricted antigens, and in particular HLA-A2 restricted antigens, as immunogenic compositions for treating and/or preventing breast cancer in an individual. In specific aspects, PR1 peptide or a derivative thereof, or a myeloperoxidase peptide, or a cyclin E1 or E2 peptide is provided in methods and compositions for breast cancer treatment and/or prevention. Such peptides can be used to elicit specific CTLs that preferentially attack breast cancer based on overexpression of the target protein cells.

WO 2012/017033 and Saccheri, Pozzi et al. 2010 refer to infection of melanoma cells with Salmonella typhimurium which induces the up-regulation of connexin 43 (Cx43). Said up-regulation is correlated with the generation of functional gap junctions between tumor cells and dendritic cells. Tumor cells, via gap junctions, transfer pre-processed antigenic peptides to the DCs which activate cytotoxic T cells specific for the tumor antigens inducing an antitumor response in vivo.

Dendritic cells (DCs) are key players in the activation of T cells. DCs comprise a family of antigen presenting cells, including plasmacytoid and conventional (myeloid) DCs. DCs are endowed with the ability to present exogenous antigens that have not been generated within DCs for the activation of T cells, via the cross-presentation pathway. Cross-presentation is required for the initiation of effective anti-tumor T cell responses and the repertoire of presented peptides is crucial to activate T cells that will recognize and kill tumor cells. However, the antigen presentation machinery, and in particular the proteasome, differs between tumor cells and dendritic cells. A major drawback is that DCs could process and present peptides that are different from those presented by tumor cells, thus initiating a tumor-specific response that will not recognize the tumor.

Gap junctions (GJs) are channels that connect the cytoplasm of two adjacent cells (B. J. Nicholson, J Cell Sci 116, 4479 (Nov 15, 2003)). They allow the transfer of small molecules including ions, second messengers and metabolites up to 1 kDa (B. J. Nicholson, J Cell Sci 116, 4479 (Nov 15, 2003)). GJ intercellular communication (GJIC) has been shown to participate to many physiological events like cell cycle control, differentiation, cell synchronization and metabolic coordination (B. J. Nicholson, J Cell Sci 116, 4479 (Nov 15, 2003), G. Mese, G. Richard, T. W. White, J Invest Dermatol 127, 2516 (Nov, 2007).). GJs are formed by two hemichannels, called connexons, each made of six Connexin proteins. There are at least 21 Connexins most of which are tissue specific except for Connexin (Cx) 43 that is ubiquitously expressed (J. Neijssen, B. Pang, J. Neefjes, Prog Biophys Mol Biol 94, 207 (May-Jun, 2007).). Loss of GJIC is a common feature in many human tumors and can occur early during tumorigenesis (T. J. King, J. S. Bertram, Biochim Biophys Acta 1719, 146 (Dec 20, 2005)., M. Mesnil, S. Crespin, J. L. Avanzo, M. L. Zaidan-Dagli, Biochim Biophys Acta 1719, 125 (Dec 20, 2005).). Recently, GJs have been shown to play a prominent role also in the immune system (J. Neijssen, B. Pang, J. Neefjes, Prog Biophys Mol Biol 94, 207 (May-Jun, 2007).). They are required for B and T cell differentiation, antibody secretion by B cells, T regulatory cell activity (T. Bopp et al., J Exp Med 204, 1303 (Jun 11, 2007).) and dendritic cell activation (E. Oviedo-Orta, W. Howard Evans, Biochim Biophys Acta 1662, 102 (Mar 23, 2004)., H. Matsue et al., J Immunol 176, 181 (Jan 1, 2006).). GJs are also involved in antigen cross-presentation by allowing the spreading of small linear peptides (up to 16 amino acid long) between neighboring cells (J. Neijssen et al., Nature 434, 83 (Mar 3, 2005).), including apoptotic cells (B. Pang et al., J Immunol 183, 1083 (Jul 15, 2009).). In absence of cell-cell contact GJ channels exist in form of hemichannels at nonjunctional membranes. Connexin hemichannels (CxHcs) in the plasma membrane are in a closed conformation under resting conditions but can be opened under the influence of stimuli such as low extracellular Ca2+, membrane depolarization, mechanical membrane stress and metabolic inhibition (Quist, Rhee et al. 2000) (Stout, Costantin et al. 2002) (Parpura, Scemes et al. 2004) (Cherian, Siller-Jackson et al. 2005).

It is still felt the need of a method providing the release in the culture medium of tumor cells, of peptides processed by the tumor proteasome with no HLA restriction and no risk of contaminating cancer cells, which could be loaded directly on DCs from the outside of the cell on surface MHC molecules.

### DESCRIPTION OF THE INVENTION

Inventors have surprisingly found that infection of tumor cells with Salmonella typhimurium induces the opening of connexin hemichannel allowing the release of pre-processed antigen peptides in the supernatant. The supernatant can be collected and used as antitumor vaccine. Inventors found that said supernatant includes poorly represented peptides including TEIPP ('T-cell epitopes associated with impaired peptide processing').

Therefore, inventors shown that by means of bacterial infection it is possible to open Cx43 hemichannels through which antigenic peptides can be released in the extracellular milieu. These peptides have been processed by the tumor proteasome and, unexpectedly, they include besides MHC class I peptides, also MHC class II peptides for both CD4 and CD8 activation as well as non-classical MHC molecules (HLA-E).

Human Leukocyte Antigen (HLA)-E is a low-polymorphic non-classical HLA class I molecule which plays a crucial role in immune surveillance by presentation of peptides to T and natural killer (NK) cells.

By including MHC class II peptides, the released peptides are able to induce an humoral response. In addition, these peptides can be loaded on DCs from the outside of the cell on surface MHC molecules. This allows to bypass the TAP transporter and consequently allows to present also peptides that would never be presented using the classical processing pathway such as TEIPP peptides.

Therefore, it is an object of the invention a method for obtaining a cell culture supernatant or a fraction thereof comprising a specific tumor antigen peptide repertoire comprising the steps of:
a) exposing in suitable conditions a tumor cell culture expressing said specific tumor antigen peptide repertoire to at least one Pattern Recognition Receptor (PRR) agonist and/or to one inflammatory cytokine to increase the opening of connexin-hemichannels (CxH);
b) collecting the cell culture supernatant; and
c) optionally obtaining a fraction of said supernatant,
wherein said supernatant comprises MHC class I and MHC class II peptides and non-classical MHC molecules (HLA-E).

Another object of the invention is a method for obtaining a specific tumor antigen peptide repertoire loaded and/or activated dendritic cell comprising the steps of:
a) exposing in suitable conditions a tumor cell culture expressing said specific tumor antigen peptide repertoire to at least one Pattern Recognition Receptor (PRR) agonist and/or to one inflammatory cytokine to increase the opening of connexin-hemichannels (CxH);
b) culturing dendritic cells with the cell culture supernatant, or a fraction thereof or with purified peptides from the cell culture supernatant, to get specific tumor antigen peptide repertoire loaded and/or activated dendritic cells; and
c) optionally purifying said specific tumor antigen peptide repertoire loaded and/or activated dendritic cells,
wherein step a) and b) are performed simultaneously or in sequence.

Another object of the invention is a method for obtaining an activated tumor antigen-specific CTL comprising the steps of:
a) exposing in suitable conditions a tumor cell culture expressing said specific tumor antigen peptide repertoire to at least one Pattern Recognition Receptor (PRR) agonist and/or to one inflammatory cytokine to increase the opening of connexin-hemichannels (CxH);
b) co-culturing dendritic cells with the cell culture supernatant, or a fraction thereof or with purified peptides from the cell culture supernatant, to get activated tumor antigen-specific CTL,
wherein step a) and b) are performed simultaneously or in sequence.

In the method for obtaining activated tumor antigen-specific CTL, dendritic cells are preferably incubated with a sample of CD8+ T cells isolated from a donor.

Preferably, the dendritic cells are autologous or HLA-compatible or -semi-compatible allogenic dendritic cells.

In a preferred embodiment of the invention, the inflammatory cytokine is gamma-IFN.

The tumor cell is preferably an established tumor cell line, or a combination of tumor cell lines expressing a specific tumor antigen peptide repertoire or a tumor cell isolated by a tumor affected subject. Said subject may be human or animal, preferably human.

Preferably, the tumor cell derives from solid or non-solid tumors, including melanoma, lung carcinoma, colorectal adenocarcinoma, prostate adenocarcinoma, sarcoma, osteosarcoma, leukemia and T cell-lymphoma and the said specific tumor antigen peptide repertoire is specific for said tumor.

In a preferred aspect, the PRR agonists are Gram-negative, preferably belonging to the *Salmonella* genus, more preferably to non virulent strains of *Salmonella* genus, or Gram-positive bacteria or components thereof.

Preferably, gram negative bacteria components are LPS and/or flagellin or Gram positive bacteria component is Lipoteichoic acid (LTA).

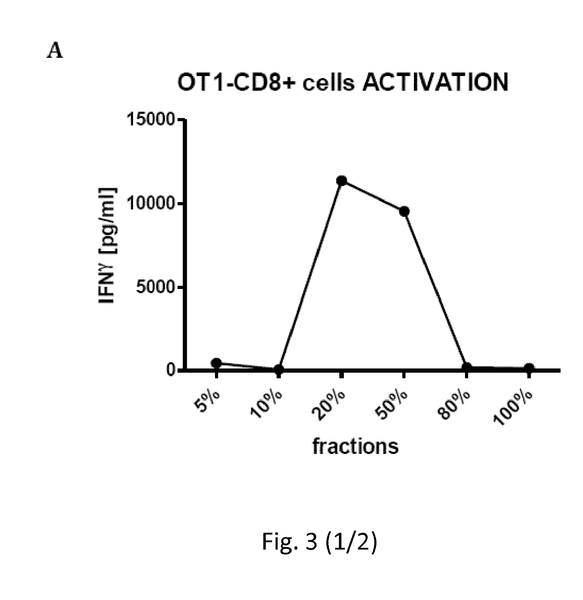

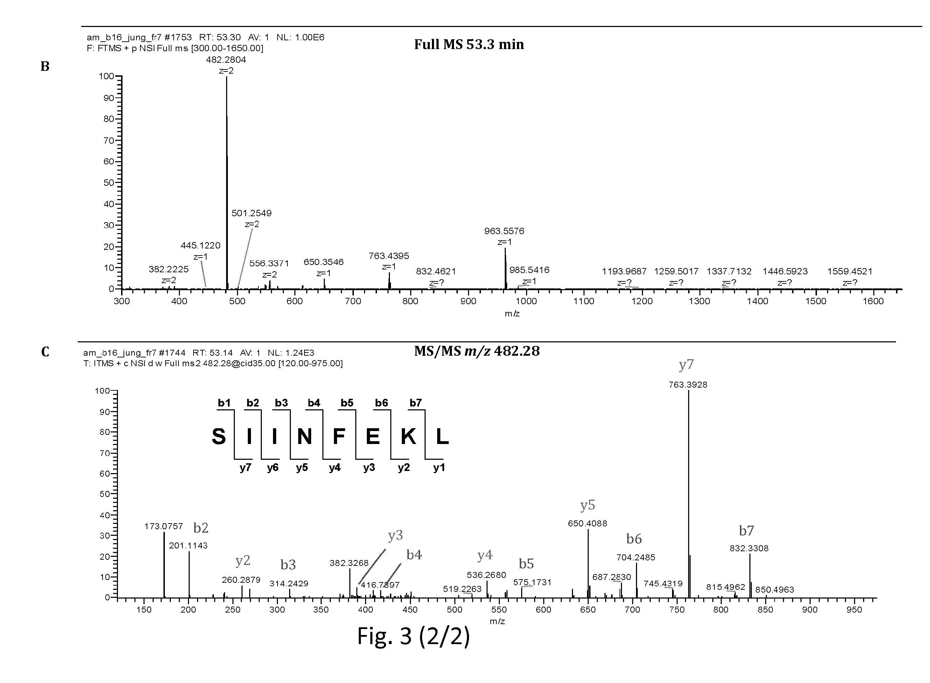

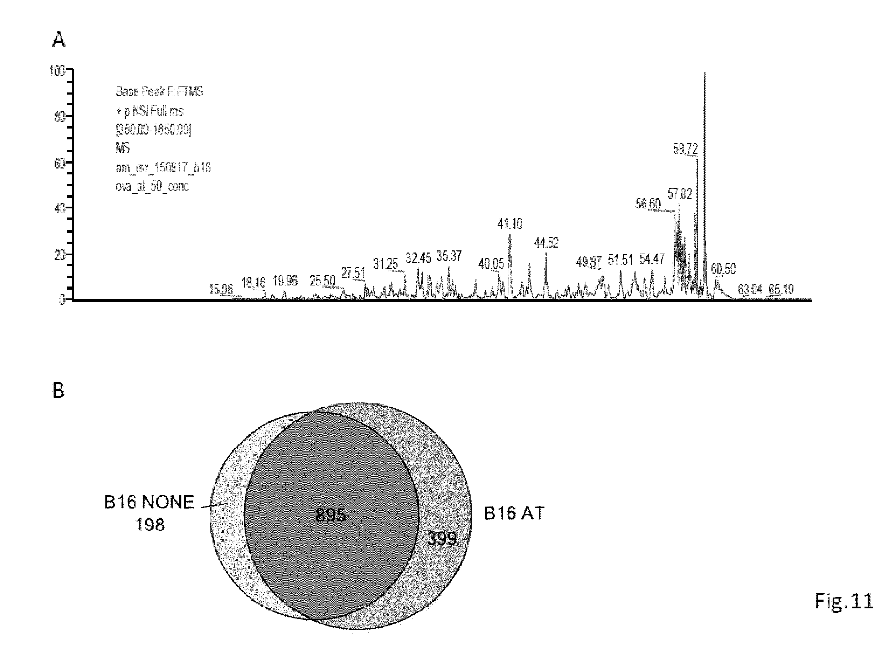

A further object of the inventions is a supernatant or a fraction thereof obtainable by the method as defined above. Preferably, the supernatant or a fraction thereof comprises peptides characterized through mass Spectrometry analysis by at least one of the pics selected from the pics represented in figures 3 and/or 11.

In a preferred embodiment of the invention, the supernatant as above defined or a fraction thereof is used in combination with a therapeutic agent, e.g. at least one antibody and/or chemotherapeutic and/or immune checkpoint inhibitor.

The supernatant as above defined, or a fraction thereof or the peptides purified by said supernatant, are preferably for use as a medicament, more preferably for use in the prevention and/or treatment of tumors, even more preferably as a tumor immunotherapeutic agent or as tumor vaccine.

A further object of the invention is a specific tumor antigen peptide repertoire loaded and/or activated dendritic cells obtainable by the above defined method preferably used in combination with a therapeutic agent, e.g. at least one antibody and/or chemotherapeutic and/or immune checkpoint inhibitor.

Another object of the invention is the specific tumor antigen peptide repertoire loaded and/or activated dendritic cells as above defined for use as a medicament, preferably for use in the prevention and/or treatment of tumors, more preferably as a tumor immunotherapeutic agent or as tumor vaccine

A further object of the invention is an immunogenic composition or vaccine comprising the supernatant as above defined, or a fraction thereof or the peptides purified by said supernatant and/or at least one specific tumor antigen peptide repertoire loaded and/or activated dendritic cell as above defined and at least one pharmaceutically acceptable carrier and/or adjuvant.

The specific tumor antigen peptide repertoire loaded and/or activated dendritic cell of the invention may be administered to a subject in suitable amounts by conventional administration routes, such as intradermal, also at multiple administration dosages, i.e. at weekly intervals, for tumor treatments. The methods of the invention are preferably in vitro or ex vivo methods.

Objects of the invention are also the tumor antigen-specific CTL as above defined, preferably used in combination with a therapeutic agent, e.g. at least one antibody and/or chemotherapeutic and/or immune checkpoint inhibitor, and said CTL for use as a medicament, preferably for use in the prevention and/or treatment of tumors, more preferably as a tumor immunotherapeutic agent or as tumor vaccine

Tumor cells present specific tumor antigen peptide repertoire derived either from tumor associated antigens or by proteins expressed also in non tumor cells that are specifically cleaved in the tumor cell, as i.e. described in Mocellin S, Mandruzzato S, Bronte V, et al. Part I: Vaccines for solid tumours. Lancet Oncol 2004;5:681-9.

An antigen loaded DC is a well known definition for the skilled person, and refers also to antigen degradation and peptide loading onto MHC molecules occurring intracellularly in Antigen Presenting Cells (APCs, such as dendritic cells). CD8+ and CD4+ T cells expressing clonally distributed receptors recognize fragments of antigens (peptides) associated with MHC class I and II molecules, respectively (Guermonprez P, Valladeau J, Zitvogel L, et al. Antigen presentation and T cell stimulation by dendritic cells. Annu Rev Immunol 2002;20:621-67).

As to the meaning of activated DCs, a well known definition for the skilled person, the dendritic cell matures into a highly effective antigen-presenting cell (APC) and undergoes changes that enable it to activate antigen-specific lymphocytes that it encounters i.e. in the lymph node. Activated dendritic cells secrete cytokines that influence both innate and adaptive immune responses (Immunobiology: The Immune System in Health and Disease. 5th edition. Janeway CA Jr, Travers P, Walport M, et al. New York: Garland Science; 2001).

Pattern recognition receptors (PRRs) refer to germline-encoded receptors that recognize molecular structures that are broadly shared by pathogens, known as pathogen-associated molecular patterns (PAMPs, Kawai T, Akira S. Toll-like receptors and their crosstalk with other innate receptors in infection and immunity. Immunity 2011;34:637-50).

A PRR agonist refers to a compound (either natural or synthetic) that binds to PRR and triggers a response.

The subject to be treated may be a human or an animal.

The therapeutic agent combined with the supernatant or a fraction thereof, or with the specific tumor antigen peptide repertoire loaded and/or activated dendritic cells or with the tumor antigen-specific CTL as above defined may be at least one immune checkpoint inhibitor (e.g. anti-PDL1, anti-PD1, anti-CTLA4, such as ipilimumab (Yervoy; Bristol-Myers Squibb), nivolumab (Opdivo; Bristol-Myers Squibb/Ono Pharmaceuticals), and pembrolizumab ((Keytruda; Merck & Co.), MEDI4736 (AstraZeneca) and MPDL3280A (Roche/Genentech)) (Webster RM. The immune checkpoint inhibitors: where are we now? Nature Reviews Drug Discovery 13, 883-884 (2014)). Dendritic cells can be obtained from any source and may be autologous or allogeneic. As used herein, a cell that is "autologous" to a subject means the cell was isolated from the subject or derived from a cell that was isolated from the subject.

As used herein, a "tumor antigen peptide repertoire loaded DC " has been contacted with the tumor cell culture supernatant under conditions that allow the DC to present peptides derived from the tumor cell supernatant in the context of MHC molecules on the cell surface.

As used herein, an "immunogenic composition" is a composition which is capable of stimulating an immune response to one or more antigens in the composition when administered to a subject. A non-limiting example of an immunogenic composition described herein is a vaccine (e.g., a DC-based vaccine), e.g., for the treatment of cancer.

As used herein, the phrase "pharmaceutically acceptable" refers to molecular entities and compositions that are generally believed to be physiologically tolerable and do not typically produce an allergic or similar untoward reaction, such as gastric upset, dizziness and the like, when administered to a human.

The composition of the invention may contain a therapeutically effective amount of DCs.

A "therapeutically effective amount" means the amount of a compound (or, e.g., cells, e.g., DCs) that, when administered to a mammal for treating or preventing a state, disorder or condition, is sufficient to effect such treatment or prevention. The "therapeutically effective amount" will vary depending on the compound or cells, the disease and its severity and the age, weight, physical condition and responsiveness of the mammal to be treated.

The pharmaceutical composition can be chosen on the basis of the treatment requirements. Such pharmaceutical compositions according to the invention can be administered in the form of tablets, capsules, oral preparations, powders, granules, pills, injectable, or infusible liquid solutions, suspensions, suppositories, preparation for inhalation.

A reference for the formulations is the book by Remington ("Remington: The Science and Practice of Pharmacy", Lippincott Williams & Wilkins, 2000).

The dendritic cells of the present invention may be provided in a pharmaceutical preparation. Said preparation may be employed in the treatment or prevention of cancer in an individual, virtually suffering from any type of solid and blood cancer. The DCs of the invention may be weekly inoculated preferentially but not exclusively intradermally, in a dose of at least 10 millions DCs. The therapy may be made up of a number of injections comprised between 2 and 20. The product is preferentially but not exclusively stored in 10% glycerol, thawed for a max of 15' at RT and immediately administered.

According to a preferred embodiment of the present invention the administration of the product can be accompanied by the administration of immunostimmulatory agents and/or adjuvants. Its usage is as well suitable in concomitance with chemotherapy as well as during the pauses between chemotherapic cycles.

The pharmaceutical compositions as above defined may be administered in a single dosage.

The expert in the art will select the form of administration and effective dosages by selecting suitable diluents, adjuvants and / or excipients.

The exposure of the tumor cell culture to the PRR or inflammatory cytokine may be obtained with any methods known to the skilled man. The suitable conditions of the exposure will be chosen so that the opening of CxH is increased.

In a preferred embodiment the connexins are connexin 43 (encoded e.g. by Mus musculus: gene ID: 14609; Homo sapiens: gene ID: 2697) and/or connexin 40 (encoded e.g. by Mus musculus gene ID: 14613; Homo sapiens gene ID: 2702), and/or connexin 45, (encoded e.g. by Mus musculus gene ID: 14615; Homo sapiens gene ID: 10052) and/or connexin 47 (encoded e.g. by Mus musculus gene ID: 118454; Homo sapiens gene ID: 57165), and/or connexin 50 (encoded e.g. by Mus musculus gene ID: 14616 and Homo sapiens: gene ID: 2703) or orthologous, allelic variants or isoforms thereof.

The term "fraction thereof" referred to the supernatant, also includes at least one peptide purified from the supernatant.

In the context of the present invention the term "tumor" includes solid or non-solid tumors, comprising melanoma, lung carcinoma, colorectal adenocarcinoma, prostate adenocarcinoma, sarcoma, osteosarcoma, leukemia and T cell-lymphoma.

The present invention will be described through non-limitative examples, with reference to the following figures:

### FIGURE LEGENDS

**Figure 1****.** Cx43 expression in B16 cells. "-" are cells not infected. "AT" is the attenuated strain of Salmonella thyphimurium SL3261AT. Vinculin is used as loading control.
**Figure 2****.** Measure of ATP extracellular concentration from uninfected or infected B16 ("AT") and B16 OVA cells treated or not with the gap-junction blocker heptanol.
**Figure 3****.** Identification of murine endogenous peptide release into the SN by Mass Spectrometry **A)** IFN-gamma secretion by OT-I CD8 T cells activated by D1 dendritic cells loaded with the indicated SN derived fractions
   Full nLC-ESI spectrum [300-1650Da] at 53.3 min of B16 OVA-derived SUP. B) JUNG (sequence: SIINFEKL [SEQ ID NO:1]) is mostly detected as double charge m/z=482.28 z=2. C) nLC-ESI-MS/MS spectrum confirmed JUNG identity (m/z 482.28, z = +2).
**Figure 4****.** Evaluation of stability of HLA molecules on the surface of T2 cells incubated with supernatant obtained from uninfected or infected SK-mel 24 and HT29 cells treated or not with heptanol. Stabilization of MHC class I complex on the surface of T2 cells indicate the presence of exogenous peptides in supernatant obtained from uninfected or infected SK-mel 24 and HT29 cells treated or not with heptanol. Control: T2 incubated with peptide Mart-1 (26-35); MFI is the mean of fluorescence intensity; *p<0.05, **p<0.01, ***p<0.001.
**Figure 5****.** IL-2 production after coculture of OVA-specific T cells with murine DCs loaded with supernatant (SN) obtained from infected (AT) or not B16 or B16 OVA cells. Negative Control: DCs (D1) alone and OVA-specific T cells alone (B3Z). Positive control: DCs loaded with OVA peptide (257-264).
**Figure 6****.** Cx43 expression in SK-mel24. TY means Salmonella TY21a-infected cells.
**Figure 7****. a)** IFN-gamma production after coculture of CTLs Mart-1 specific with moDCs loaded with supernatant obtained from SK-me124 or 624.38 treated as indicated. Control: moDCs loaded with Mart-1 (26-35). **b)** Same as in a) but dendritic cells are purified ex-vivo (PDC, Plasmacytoid dendritic cells).

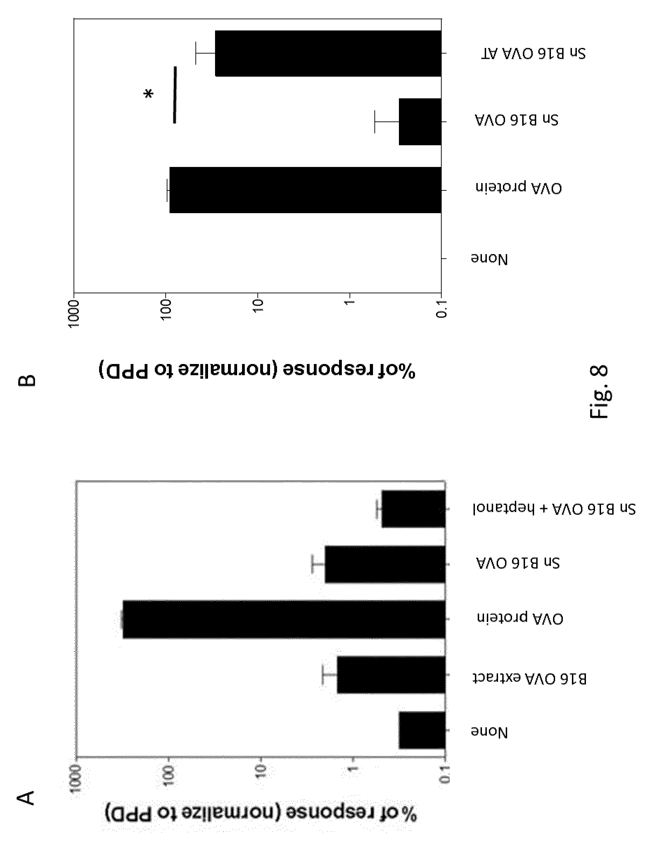

**Figure 8****.** Mice were immunized in CFA (complete Freund's adjuvant) with the indicated products. as indicated. In figure A immunization was performed with OVA protein, B16 OVA extract, supernatant (SN) from B16 OVA cells in absence or presence of the gap junction inhibitor heptanol. In figure B immunization was performed with OVA protein, SN from B16 OVA or SN from Salmonella infected B16 OVA (B16 OVA AT). After 7-9 days lymph nodes cells were re-stimulated in vitro with PPD (Tuberculin purified protein derivate) or OVA protein and IFN-gamma secretion was measured after 72 hours of culture. *p< 0.05.
**Figure 9****.** Antibody response to Salmonella SL3261AT peptides (A) and B16 tumor peptides (B). MG132 is a proteasome inhibitor; IFA is Freund's incomplete adjuvant.
**Figure 10****.** Tumor growth in mice vaccinated with DCs loaded with supernatant from uninfected (B16) or Salmonella infected B16 cells (B16 AT). DCs loaded with supernatant vaccine are protective against melanoma. Control: DCs not loaded. *p<0.05.
**Figure 11** Mass Spectrometry analysis of murine endogenous peptides differentially released into SN of B16 Salmonella infected cells. A) Full MS spectrogram of a fraction of bacteria treated B16 cells-derived-supernatant. B) Venn Diagram resulted by the analysis of the protein associated to the detected peptides comparing bacteria treated versus untreated B16 cells-derived-supernatant.
**Figure 12****.** Canine osteosarcoma cell culture A) OSA cells analyzed by optical microscope, B) OSA cells stained red for phalloidin (cytoplasmic staining) and blue with DAPI (nuclear staining), C) alkaline phosphatase staining.
**Figure 13****.** Cx43 expression in canine osteosarcoma cells (OSA), infected or not with Salmonella Ty21 a.

### EXAMPLE

### MATERIAL AND METHODS

### Mice, cell lines and bacterial strain

Six-week-old female C57BL/6J mice were purchased from Charles River and maintained in specific pathogen-free animal house. The cell lines used in present study were murine melanoma B16F10 (called throughout B16) were cultured in RPMI 1640 medium supplemented with 10% fetal serum bovine, 2mM Glutamine, 100 U/ml Penicillin,100µg/mL Streptomycin and 50 µM 2-Mercaptoethanol (complete RPMI).

The murine melanoma B16F10-OVA (called throughout B16 OVA) were cultured in Iscove's Modified Dulbecco's Medium (IMDM) supplemented with 10% fetal serum bovine, 2mM Glutamine, 100 U/ml Penicillin, 100µg/mL Streptomycin and 50 µM 2-Mercaptoethanol,100µg/mL Hygromycin B. Primary canine osteosarcoma cells were obtained from dissociation of dog's osteosarcoma specimens. Tissues were minced with a scalper in a cell strainer. Cells were washed with DMEM (supplemented with 10% FBS, 2 mM L-Glutamine, 100 U/mL Penicillin, 100 mg/mL Streptomycin) and filtered with a cell strainer (100 µM).

The cellular suspension was centrifuged at 300 g for 10 minutes and resuspendend with 2 mL of Lysis Buffer in order to lyse red blood cells; then the cells were washed twice with complete medium and cultured. Murine dendritic cells (D1), derived from bone marrows of C57BL/6J mice were cultured in IMDM containing 10% FBS, supplemented with 30% supernatant from granulocyte macrophage colony-stimulating factor-producing NIH-3T3 cells.

The B3Z T-cells hybridoma specific for the H-2Kb restricted OVA peptide were grown in Iscove's modified Dulbecco's medium (IMDM) supplemented with 5% FBS.

C57BL/6J OT-I mice contain transgenic T cell receptor designed to recognize OVA(₂₅₇₋₂₆₄) in the context of H2Kb. Purified CD8 OT-I T cell were re-stimulated in vitro by D1 dendritic cell line previously loaded with OVA(₂₅₇₋₂₆₄) or SN of B16 OVA infected (AT) or not, in RPMI 1640 medium supplemented with 10% fetal serum bovine, 2mM Glutamine, 100 U/ml Penicillin, 100µg/mL Streptomycin.

T2 cells are an HLA-A0201 hybrid human cell line lacking TAP-2 and express low amount of MHC class I on their surface. These cells were cultured in RPMI 1640 medium supplemented with 10% fetal serum bovine, 2mM Glutamine, 100 U/ml Penicillin, 100µg/mL Streptomycin.

The bacteria, Salmonella typhimurium SL3261AT is an aroA metabolically defective strain on SL1344 background and is grown at 37 °C in Luria broth (LB).

Vivofit® (Thyphoid vaccine live oral Ty21a) is a live attenuated vaccine containing the attenuated strain of Salmonella enteric serovar thyphi Ty21a and is grown at 37 °C in Luria broth (LB).

### In vitro infection with bacteria and supernatant production

Single bacterial colonies were grown overnight and restarted the next day to reach an absorbance at 600 nm ranging between 0,550 and 0,650 corresponding to 0,550-0,650 x10₉ colony -forming units (CFUs)/mL. Tumor cells were incubated with or without Gap Junctions blocker, Heptanol (1mM) for 1 hour and half at 37 °C. Then, cells were incubated with bacteria for 1 hour and half at 37 °C at a cell-to-bacteria ratio of 1:50, in the appropriate medium without antibiotics. After incubation, the cells were washed and incubated at 37 °C in medium supplemented with gentamicin (50µg/mL) for 2 hours. The cells were washed twice and incubated overnight with medium supplemented only with gentamicin in order to kill extracellular bacteria. The next day, the cells were centrifuged at 2000 rpm for 5 minutes; the supernatant was collected and filtered with 70 µM cell strainer. The supernatant was freeze-dried.

### Mart-1-specific CTL generation

To generate peptide-specific CTLs from PBMC, 106 PBMC HLA-A2+ were cultured in 1 mL of RPMI 1640 medium (supplemented with 5% human serum,2mM L- Glutamine, 100U/mL Penicillin, 100 µg/mL Streptomycin, 10 µg/mL Gentamicin,10 µg/mL beta-mercaptoethanol and 1% nonessential amino acids) in 24-well-plates. To this, 2x106 Mart-1-pulsed and irradiated (10 Gy) PBMC (HLA-A2) were added as antigen presenting cells in the same medium supplemented with 100 U/mL IL-2. To pulse PBMC they were incubated for 90 minutes at 37°C in RPMI supplemented with 50 µM of peptide. After incubation, cell were washed twice and irradiated before mixing with the responding cells. The cells were stimulated at intervals of 10 days with irradiated peptide-pulsed autologous PBMC and 100 U/mL IL-2.

### Adenosine 5'-triphosfate (ATP) bioluminescent assay

Adenosine 5'- triphosphate (ATP) Biolumescent Assay (CellTiter-Glo Luminescent cell viability Assay, Promega) allow to measure the quantity of ATP containing in the samples. ATP is consumed and light is emitted when firefly luciferase catalyzes the oxidation of D-luciferin. Briefly, 100 µL of Assay Mix solution were added to a reaction vial for 3 minutes at room temperature; then rapidly were added 100 of sample diluent, mixed and immediately measured the amount of light produced. The final value is proportional to the amount of ATP in the sample.

### SN preparation, functional assay and Mass Spectrometry

Supernatant (SN) from B16 or B16-OVA untreated or Salmonella treated cells were treated as follow. Proteins were removed from the SN through a Trichloroacetic acid (TCA)-based protein precipitation procedure. In detail, samples were incubated with TCA (13% final concentration) for 5min at -20°C and then for 5 hours at 4°C. Samples are then ultra-centrifuged at 15000g for 15min; peptides-enriched SN was collected and the pellet was discarded.

Products were loaded on the C18 matrix and washed with 0.1% Formic Acid. Finally, peptides are eluted with increasing percentages of Acetonitrile (5%, 10%, 20%, 50%, 80%).

Acetonitrile was removed by speed vacuum centrifuge.

Fractions were analyzed by matrix-assisted laser desorption / ionisation - time of flight mass spectrometry (MALDI-TOF MS). Full nLC-ESI spectrum [300-1650Da] at 53.3 min was analyzed to confirm SIINFEKL (SEQ ID NO:1) identity.

The presence of functional SIINFEKL (SEQ ID NO:1) peptide, OVA(₂₅₇₋₂₆₄), was assessed by a functional assay using OT-I CD8 cells as describe in Materials and Methods.

### T2 assay for peptide binding

The T2 binding assay is based upon the ability of peptides to stabilize the MHC class I complex on the surface of T2 cells.

T2 cells were incubated overnight at 37 °C at 2x105 cells / well in FCS-free RPMI medium with 100 µL of supernatant or MART-1 peptide (1 µM and 10 µM) as a positive control.

The next day, the cells were washed with FACS buffer (PBS, 0.1% sodium azide,5% fetal bovin serum) and incubated for 10 min with blocking buffer (200 µg/mL mouse IgG in FACS buffer). Then, the cells were incubated for 20 minutes at +4 °C with BB7.2, an HLA-A2 conformation specific mouse antibody. The cells were washed twice with FACS buffer and fixed in paraformaldehyde for later acquisition by Accuri C6 Flow Cytometer (BD).

### Immunofluorescence staining

OSA cells were washed twice with PBS and incubated for 30 minutes at room temperature with blocking buffer (PBS + 0.03% of Tryton + 2% FBS). Then, cells were incubated with Ab anti-phalloidin for 30 minutes at room temperature. The cells were washed twice with PBS and examined by fluorescent microscope.

### Alkaline Phosphatase Staining

OSA cells were washed twice with PBS and incubated with paraformaldeide (1%) for 10 minutes at room temperature. The cells were washed and incubated with NBT/BCIP solution for 1 hour at room temperature. Then, the cells were washed with PBS and examined microscopically.

### In Vivo immunization with supernatant

Mice were immunized subcutaneusly with 100 µL of emulsion with supernatant containing released peptides and either Freund's Complete Adjuvant (CFA) or Freund's Incomplete Adjuvant (IFA).

Nine days after the immunization, mice were killed, popliteal lymph nodes were smashed and cells were plated in flat-bottom 96-well plates and stimulated with PPD (3 µg/mL), OVA protein (30 µg/mL), OVA peptide 323-339 (3 µg/mL) or OVA peptide 257-264 (3 µg/mL). After 72 hours, the supernatant was collected and IFN-y production was measured by ELISA.

To evaluate antibody response mice were killed four weeks after immunization, and serum was collected. The antibody titer to Salmonella and B16 antigens was evaluated by ELISA.

### In Vivo vaccination with DC loaded supernatant

DC1 dendritic cells, matured with LPS (1 µg/mL), were loaded with supernatant containing released peptides for 4 hours at 37 °C. After incubation, the cells were washed twice and subcutaneously injected in the right flank of mice (on days 0 and 4). On 21 day 10⁵ B16 cells were subcutaneously injected in the left flank.

### RESULTS

### Opening of connexin hemichannels by Salmonella Infection of melanoma cells

In the work of Saccheri et al (Saccheri, Pozzi et al. 2010) and in WO 2012/017033, it was shown that Salmonella induces, in melanoma cells, the up-regulation of connexin 43 (Cx43), a ubiquitous protein that forms gap junctions and that is often lost during carcinogenesis.

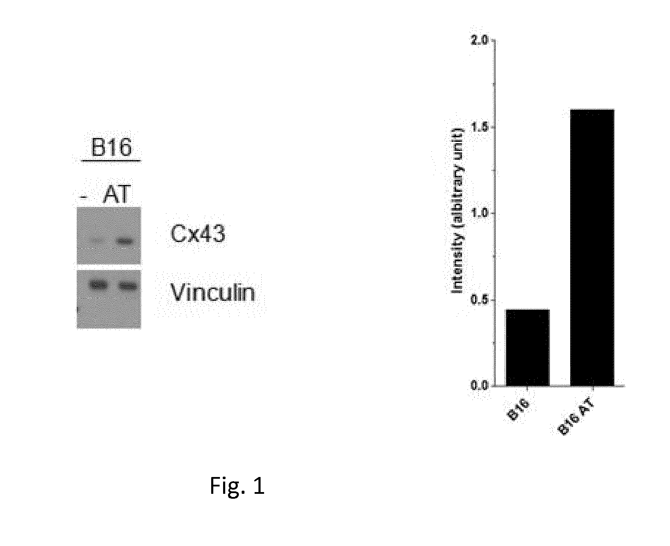

*Figure 1* confirmed that Cx43 expression was up-regulated in B16 melanoma cells after infection with the attenuated strain of Salmonella thyphimurium SL3261AT, as evaluated by Western blot analysis.

As described in the introduction, the single hemichannel that forms a gap junction in the plasma membrane is closed under resting conditions but can be induced to open under the influence of different stimuli (Saez, Retamal et al. 2005).

In order to assess whether Salmonella is able to stimulate the opening of connexin hemichannels on the surface of melanoma cells, inventors measured ATP extracellular concentration using an adenosine 5'-thriphosfate (ATP) bioluminescent assay (as described in Materials and Methods) in Salmonella treated B16 cells.

Briefly, inventors used the attenuated strain of Salmonella thyphimurium SL3261AT to infect mouse melanoma cell line B16 and the same cells expressing ovalbumin (B16 OVA), previously treated or not with the gap-junction blocker, heptanol.

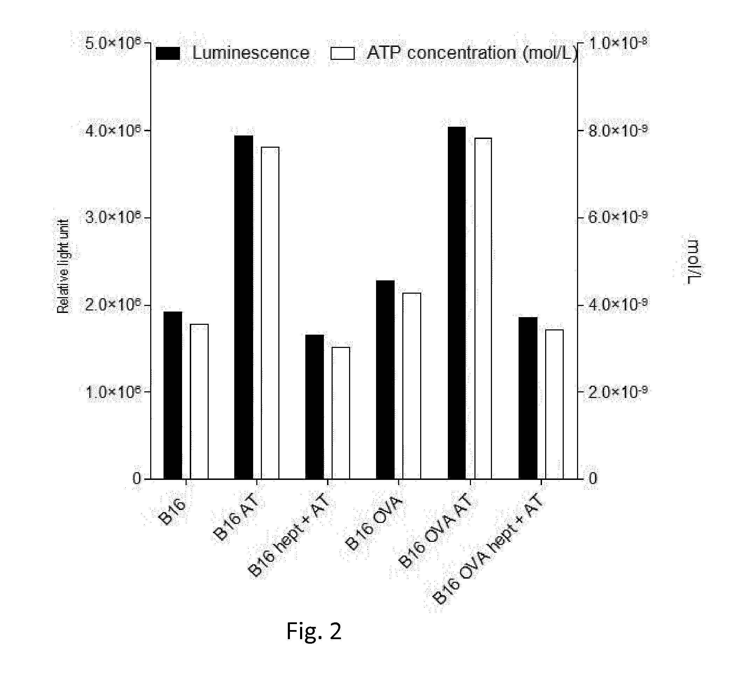

As shown in *figure* 2, Salmonella infection induces the release of extracellular ATP from both cell lines and this effect is significantly reduced by the gap-junction blocker heptanol. This result indicates that a cytoplasmatic molecule can be released by connexin hemichannel (CxH) in a Salmonella dependent manner, demonstrating the role of bacteria to stimulate the opening of hemichannels, in a condition where it induces the up-regulation of Cx43.

### Identification of released endogenous peptides functional assay and Mass Spectrometry

SIINFEKL (SEQ ID NO:1) H-2Kb restricted OVA octapeptide (OVA ₂₅₇₋₂₆₄) is know to be processed and presented by B16-OVA MHC class I molecules. To identify this prototype endogenous peptide, released in the supernatant, inventors used the attenuated strain of Salmonella thyphimurium SL3261AT (SL) to infect mouse melanoma cell line B16 and the same cells expressing ovalbumin (B16 OVA). B16 OVA derived SN treated as described in material and methods were analyzed both functionally and biochemically to detect the presence of (OVA ₂₅₇₋₂₆₄) in the derived fractions.

As shown in *figure 3a* CD8 OT-I T cells activation measured, as IFN-gamma released, is mainly present in SN fractions 20% and 50%, which were further analyzed by mass spectrometry.

Full MS, *figure 3b**,* and full nLC-ESI, *figure 3c**,* spectra [300-1650Da] at 53.3 min of B16 OVA-derived SN was performed and analysis of nLC-ESI spectrogram confirm the SIINFEKL (SEQ ID NO:1) identity demonstrating the release of a processed endogenous peptide in SN.

### Gap-junction hemichannel dependent tumor peptides release by Salmonella-infected tumor cells

Inventors continued their investigation asking whether cytoplasmic peptides could be transferred in a CxH dependent manner by *Salmonella* infected tumor cells to the extracellular milieu.

In order to investigate the release of MHC class I peptides by *Salmonella* infected tumor cells, inventors exploited the ability of exogenous peptides to stabilize the MHC class I complex on the surface of T2 cells. T2 are an HLA-A0201 hybrid human cell line lacking TAP-2 (transporter-associated with antigen processing) and consequently are defective in loading class I molecules with antigenic peptides generated in the cytosol. This leads to very unstable MHC class I molecules on the cell surface. The association of exogenously added peptides stabilizes surface expression of HLA molecules, recognizable by specific anti-HLA-A0201 antibody.

Briefly, inventors infected human melanoma cells SKmel-24 and colorectal adenocarcinoma cells HT29, with Vivofit®, an oral typhoid vaccine that contains live, attenuated cells of the bacteria *Salmonella enterica* serovar Thyphi (TY21a), and inventors collected the supernatant as described in Materials and Methods. T2 cells were incubated with the supernatant obtained from uninfected or infected cells treated or not with the gap junction blocker (heptanol). Surface expression of HLA-A0201 was evaluated using a conformation-specific mouse antibody, as described in Materials and Methods.

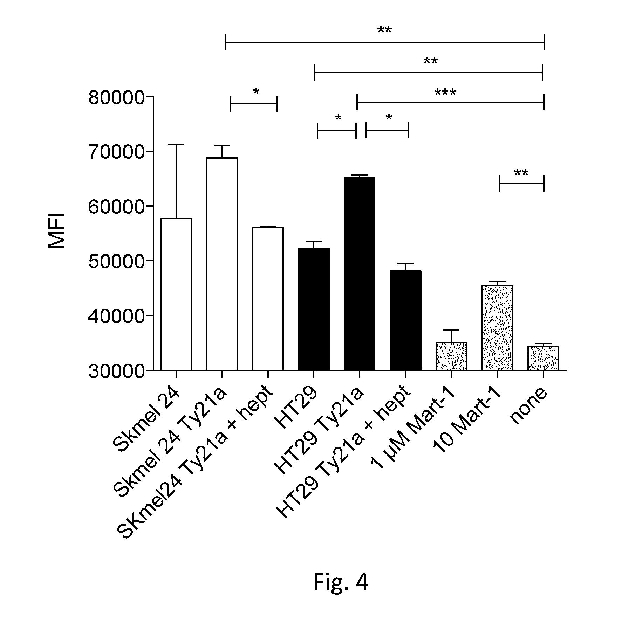

*Figure 4* shows that T2 cells incubated with Mart-1 (26-35) peptide, as positive control, display a level of HLA-A0201 mean fluorescence intensity (MFI) significantly higher than that of unloaded T2 cells (none), indicating that the presence of the exogenous peptides is capable of stabilizing MHC class I complexes on the surface of T2 cells. Incubation of T2 cells with supernatant obtained from infected tumor cells increases the MFI that is abolished by the gap junction blocker. These results demonstrate that peptides from *Salmonella* infected tumor cells are released in a CxH-dependent manner and they can bind to MHC class I molecules.

Similar results were obtained using supernatants collected from another tumor cell line (colorectal adenocarcinoma cells) suggesting that this phenomenon could be widely applied to other type of cancer cells.

Since infected tumor cells are able to release peptides in a CxH-dependent manner, inventors tested whether antigen-specific T cells could recognize those peptides. Murine dendritic cells (D1), previously incubated with the supernatant obtained from B16 cells expressing ovalbumin (B16-OVA) infected or not with Salmonella SL3261AT, were cocultured with the OVA specific-B3Z hybridoma T cells. After 72 hours, the amount of IL-2 secretion was assessed by ELISA as a measure of OVA peptide recognition.

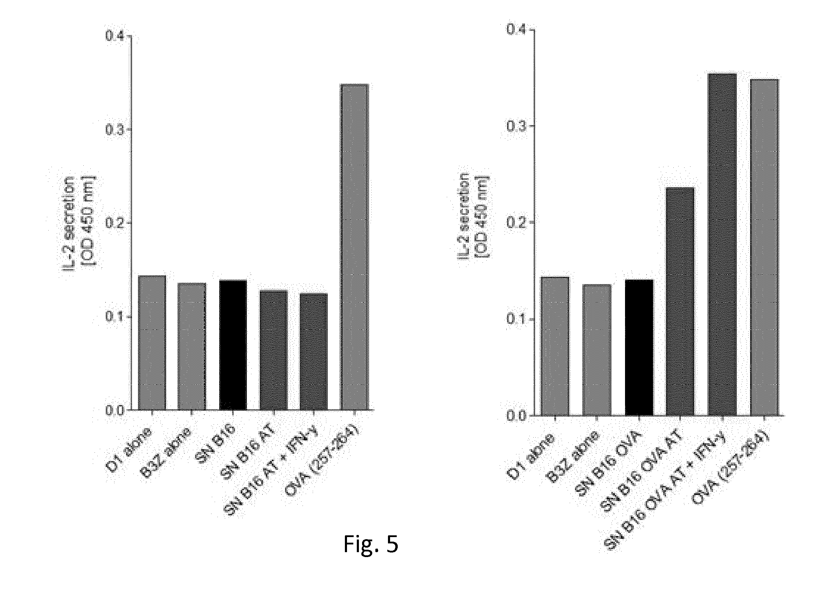

In *figure 5* it is shown that dendritic cells incubated with supernatant obtained from B16-OVA cells treated with Salmonella alone or in combination with IFN-y activate OVA- specific T cells as shown by the increase of IL-2 secretion. This result demonstrates that the antigenic peptides released by Salmonella infected tumor cells are recognized and are able to activate antigen-specific T cells.

By contrast, when dendritic cells loaded with supernatant obtained from B16 cells, were co-cultured with OVA specific T cells there was no production of IL-2, demonstrating the absence of OVA peptide and the specificity of the assay.

Based on this evidence, inventors decided to investigate the release of tumor peptides by *Salmonella* infected human tumor cell lines.

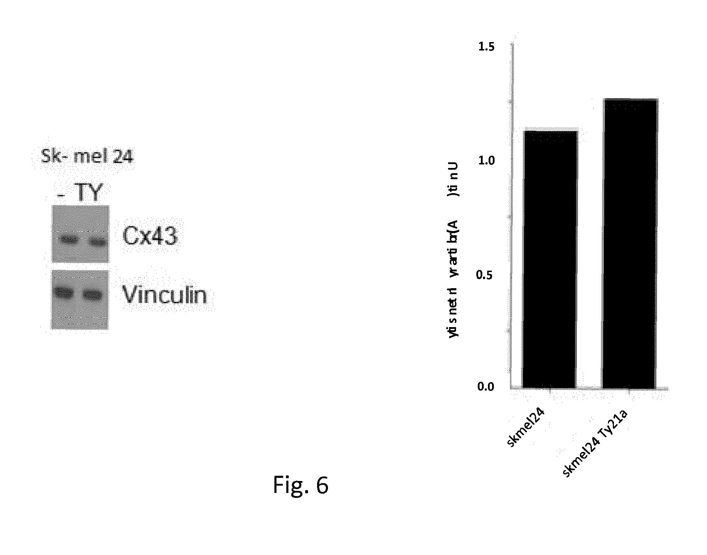

Inventors analysed, by Western blot analysis, the effect of *Salmonella* infection on Cx43 expression in human melanoma cells, SK-mel 24. As shown in the *figure* 6, Cx43 expression is high already in resting conditions and after *Salmonella* infection is slightly up-regulated.

To evaluate the release of antigenic peptides inventors infected human melanoma cells SKmel24 and 624.38 with *Salmonella* TY21a in presence or not of heptanol and the supernatant was harvested as described in Materials and Methods.

Human monocyte derived DCs differentiated *in vitro* from monocytes with GM-CSF and IL-4 (moDCs) were incubated with the supernatant, produced as indicated above, and cocultured with Mart-1 (26-35) specific CTLs (obtained as described in Materials and Methods). After 72 hours of coculture, the amount of IFN-gamma secretion was measured by ELISA.

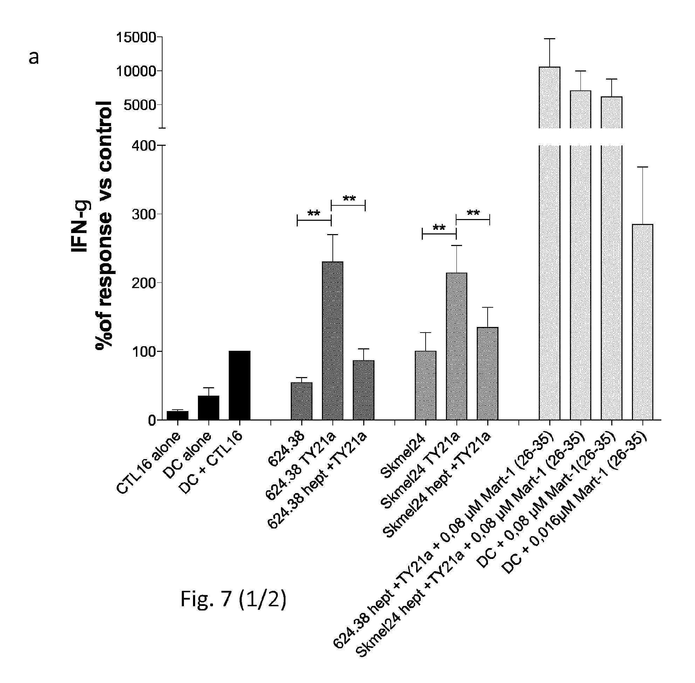

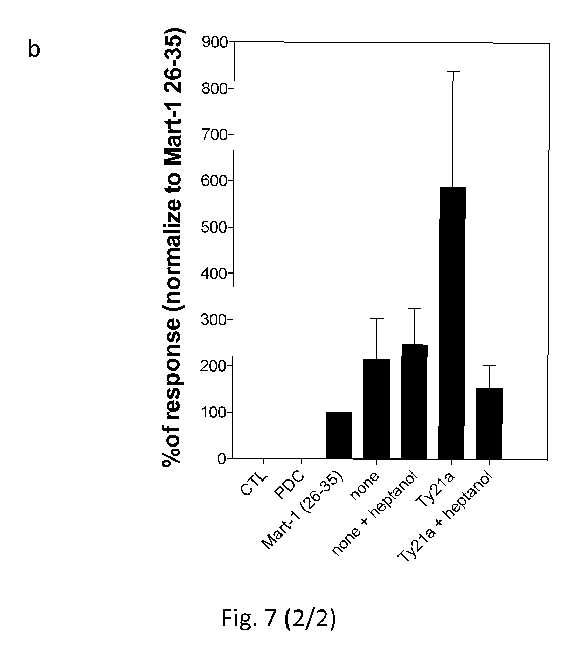

*Figure 7a* *and* *fig 7b* show respectively that moDCs or PDC incubated with supernatant of melanoma infected tumor cells, activate Mart-1 (26-35) specific human CTLs and the gap junction blocker significantly inhibits this effect. Exogenous addition of Mart-1 (26-35) peptide restored completely the response.

This result demonstrates a CxH-dependent release of tumor peptides by *Salmonella* infected human melanoma cell lines and their ability to activate tumor antigen-specific human CTLs.

### Release of tumor peptides by Salmonella infected tumor Cells induces an in vivo antitumor immune response

To investigate whether the tumor peptides released from infected tumor cells could induce an *in vivo* immune response, C57BL/6J mice were immunized in the footpad with supernatant, obtained from B16 OVA cells infected or not with Salmonella SL3261AT, emulsified with Freund's complete adjuvant (CFA).

Briefly, nine days after immunization, the popliteal lymph nodes were removed and cells were stimulated in vitro with PPD (Tuberculin purified protein derivated), as a positive control, and OVA protein. After 72 hours of incubation, the amount of IFN-gamma secretion was assessed by ELISA to evaluate the activation of immune cells.

As shown in *figure 8A**,* immunization with the supernatant obtained from B16 OVA cells induced IFN-gamma production after in vitro recall with OVA protein that was reduced by heptanol. This response is similar to that induced after immunization with B16 OVA extract. Instead, as shown in *Figure 8B*, when the mice were immunized with the supernatant obtained from Salmonella infected B16 OVA (SN B16 OVA AT), the IFN-gamma secretion was increased. These results demonstrate that peptides released by Salmonella infected tumor cells can induce an in vivo immune response and such response is CxH-dependent.

In order to identify the type of released tumor antigens, C57BL/6J mice were immunized subcutaneously with supernatant obtained from Salmonella infected or uninfected B16 cells treated or not with the gap-junction blocker (heptanol) or the proteasome inhibitor (MG132), emulsified with Freund's Incomplete adjuvant (IFA). Four weeks later inventors evaluated the amount of total IgG specific to Salmonella SL3261AT and to mouse melanoma cell line B16 by ELISA.

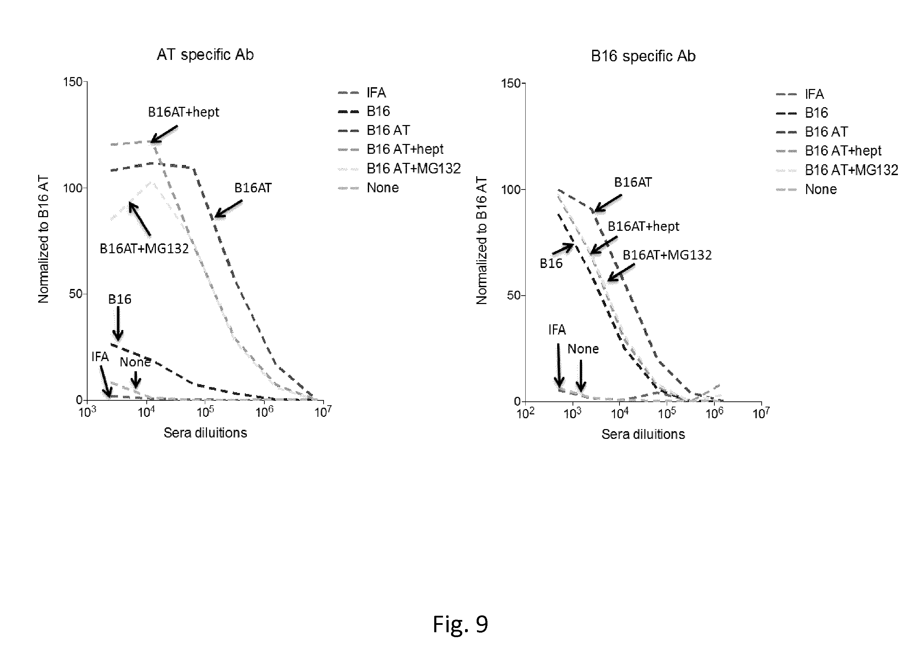

*Figure 9A* shows that immune sera from mice immunized with the supernatant obtained from infected B16 cells recognize Salmonella antigens and this response is reduced by heptanol and MG132. This data suggests that Salmonella after infection is processed and peptides are released in the supernatant through CxH, as shown by the effect of heptanol. Importantly, these peptides are partly generated by proteasome processing because the antibody response is reduced by MG132 treatment.

*Figure 9B* shows that Salmonella infection increased the release of B16 proteasome processed tumor peptides as indicated by the presence of B16 specific antibody in the serum of mice immunized with supernatant obtained from infected B16 cells.

This effect is reduced by heptanol and MG132, underlining the release of proteasome-processed tumor peptides by CxH.

Moreover, in *figure 9B**,* it was shown that an antibody response against tumor peptides was induced also in mice immunized with supernatant obtained from uninfected B16 cells. However, the titer of antibody response is significantly lower than that of Salmonella infection's group, probably due to the lower concentration of tumor peptides release in the supernatant.

These results indicate the induction of an in vivo immune response by pre-processed tumor peptides released by Salmonella infected tumor cells.

In order to investigate the effect on tumor growth induced by the released pre-processed tumor peptides, inventors decided to use dendritic cells as adjuvant.

Briefly, inventors vaccinated C57BL/6J mice with murine dendritic cells (D1) previously loaded with supernatant obtained from non infected or infected B16 cells, twice (on days 0 and 4) before the challenge with B16 cells (day 21).

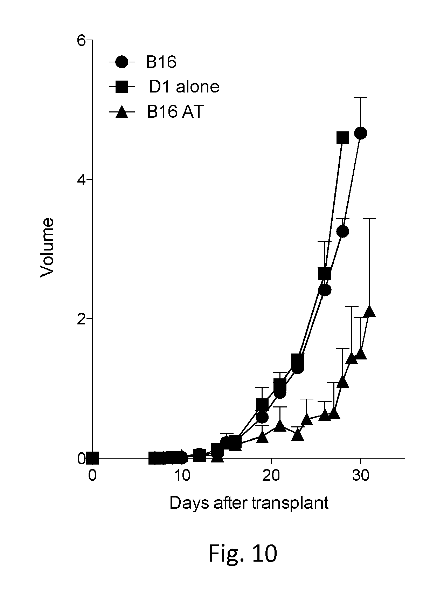

In *figure 10**,* it is shown a statistically significant delay of tumor growth in mice vaccinated with DCs loaded with supernatant obtained from Salmonella infected B16 cells (red line) and the effect was lost when DCs were loaded with supernatant obtained from uninfected B16 cells (black line). The tumor peptides released by Salmonella infected tumor cells were captured and cross-presented by dendritic cells inducing an in vivo antitumor immune response.

These data confirm that tumor peptides released by Salmonella infected tumor cells are able to induce an in vivo anti-tumor immune response.

### Mass Spectrometry analysis identify pool of endogenous peptides differentially released by B16 Salmonella infected cells

In order to assess whether endogenous peptides are differentially released in the SN by SL infection, B16 cell were infected with SL and SN recovered and treated as describe in material and methods. *Figure 11a* showed full MS spectra of B16 infected SN, and Venn diagram *figure 11b* highlight 399 protein associated to the detected and sequence peptides, that are differentially released in the SN of B16 SL-infected cells.

These data indicate that the ability to induce an in vivo anti-tumor immune response reside in a pool of immunogenic tumor peptides released by Salmonella infected tumor cells.

### Translational study of canine osteosarcoma

Starting from these preliminary results, inventors then investigated whether this strategy could be translatable to other types of tumors, testing our approach in an experimental veterinary study for the treatment of a deadly form of spontaneous canine osteosarcoma.

Tumor specimens were obtained from a Veterinary clinic and primary osteosarcoma cell lines were generated as described in Materials and Methods.

Experimental vaccine containing the supernatant collected after infection of canine osteosarcoma cells with the vaccine strain Vivofit® of Salmonella enterica serovar thyphi (TY21a) (as described in Materials and Methods) was produced for all dog patients.

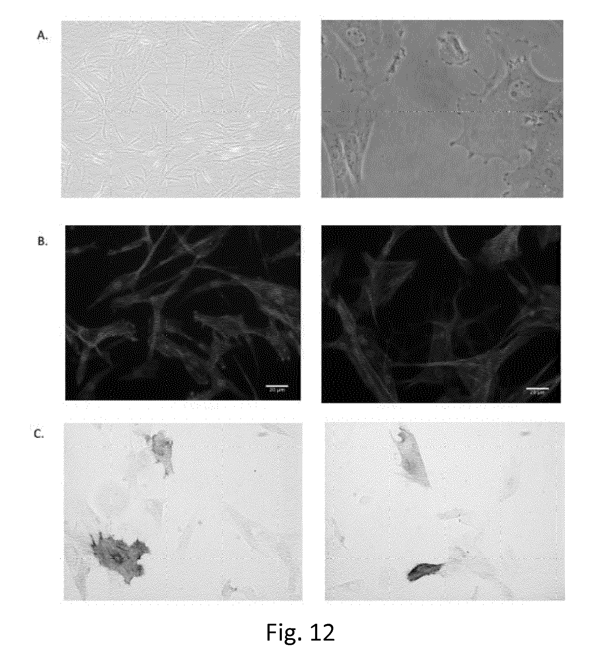

In order to induce the shrinkage of the tumor and afterwards a long lasting anti-tumor immune response, the treatment schedule includes the association of standard chemotherapy (4 cycles of carboplatin every 21 days) with experimental vaccination. Vaccination started after 2 cycle of standard chemotherapy. Two cycle of vaccination were administered intradermically, after topical application of 5% Imiquimod cream (Aldara®) on the injection site with the following modality: first cycle of 2 vaccinations at 21 days intervals and second cycle of 4 vaccinations at 30 days intervals. The patients will remain under observation for the following 24 h in the veterinary clinic. *Figure 12* shows primary canine osteosarcoma cells obtained from dog's osteosarcoma specimen. Panel A shows OSA cells analyzed by optical microscope: the cells appear adherent, mostly elongated of varying size or large pentagonal and polyhedral. There are numerous characteristic cytoplasmatic granules and vacuoles in most cells.

Panel B shows OSA cells stained with anti-phalloidin antibody to visualize actin filaments and with DAPI for nuclear counterstain. Moreover, inventors identified OSA cells by staining them for alkaline phosphatase activity (panel C).

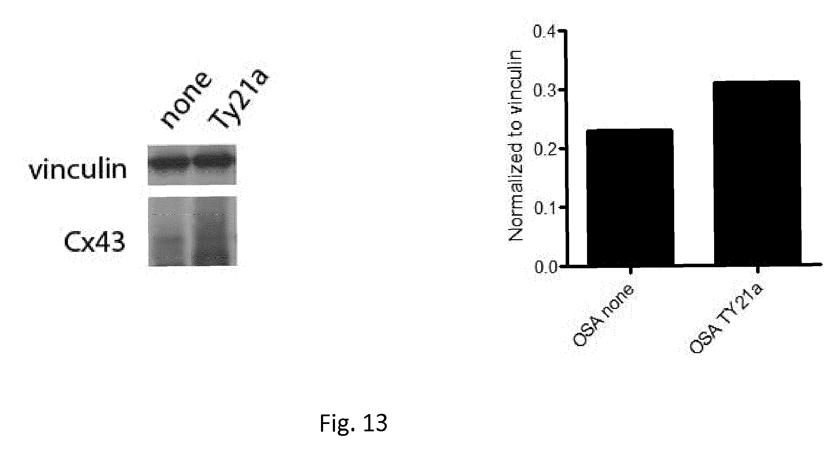

Finally, inventors characterized the effect of Salmonella on connexin 43 (Cx43) in canine osteosarcoma cells. As evaluated by Western blot analysis (*Figure 13*), Salmonella Ty21a is able to up-regulate Cx43 expression in osteosarcoma cells.

Inventors enrolled twenty osteosarcoma dog patients and five of these started the experimental therapy.

Prognosis for dogs undergoing surgery and chemotherapy is approximately 235-360 days after diagnosis. Since from one dog inventors could not obtain the specimen to generate the cell line, inventors decided to treat it using the vaccine generated from another dog in heterologous fashion. Our preliminary results (*Table I*) indicate that the dog (case 1) treated with the heterologous vaccine survived 653 days since diagnosis and died for tumor-unrelated causes and the dog (case 2) treated with autologous vaccine had survived 224 days. In addition, one dog (case 3) is still alive after 700 days of diagnosis.

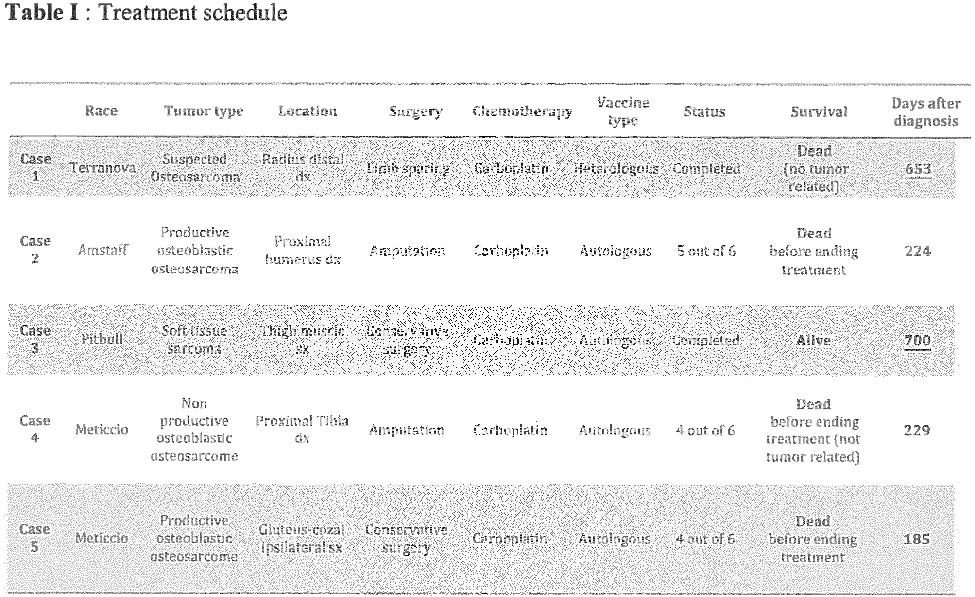

### REFERENCES

Mocellin, S. (2012). "Peptides in melanoma therapy." Current pharmaceutical designl8(6): 820-831.
Adams, S., M. A. Lowes, et al. (2008). "Lack of functionally active Melan-A(26-35)-specific T cells in the blood of HLA-A2+ vitiligo patients." The Journal of investigative dermatology128(8): 1977-1980.
Fourcade, J., Z. Sun, et al. (2010). "Human tumor antigen-specific helper and regulatory T cells share common epitope specificity but exhibit distinct T cell repertoire." Journal of immunologyl 84(12): 6709-6718.
Speiser, D. E., P. Baumgaertner, et al. (2008). "Unmodified self antigen triggers human CD8 T cells with stronger tumor reactivity than altered antigen." Proceedings of the National Academy of Sciences of the United States of America105(10): 3849-3854.
Meijer, S. L., A. Dols, et al. (2007). "Induction of circulating tumor-reactive CD8+ T cells after vaccination of melanoma patients with the gp100 209-2M peptide." Journal of immunotherapy30(5): 533-543.
Knittelfelder, R., A. B. Riemer, et al. (2009). "Mimotope vaccination--from allergy to cancer." Expert opinion on biological therapy9(4): 493-506
van Stipdonk, M. J., D. Badia-Martinez, et al. (2009). "Design of agonistic altered peptides for the robust induction of CTL directed towards H-2Db in complex with the melanoma-associated epitope gp100." Cancer research69(19): 7784-7792.
Parmiani, G., C. Castelli, et al. (2002). "Cancer immunotherapy with peptide-based vaccines: what have we achieved? Where are we going?" Journal of the National Cancer Institute94(11): 805-818.
Saccheri, F., C. Pozzi, et al. (2010). "Bacteria-induced gap junctions in tumors favor antigen cross-presentation and antitumor immunity." Science translational medicine2(44): 44ra57.
Saez, J. C., M. A. Retamal, et al. (2005). "Connexin-based gap junction hemichannels: gating mechanisms." Biochimica et biophysica acta1711(2): 215-224.

## Claims

1. A method for obtaining a cell culture supernatant or a fraction thereof comprising a specific tumor antigen peptide repertoire comprising the steps of:
a) exposing in suitable conditions a tumor cell culture expressing said specific tumor antigen peptide repertoire to at least one Pattern Recognition Receptor (PRR) agonist and/or to one inflammatory cytokine to increase the opening of connexin-hemichannels (CxH);
b) collecting the cell culture supernatant; and
c) optionally obtaining a fraction of said supernatant,
wherein said supernatant comprises MHC class I and MHC class II peptides and non-classical MHC molecules (HLA-E).

2. A method for obtaining a specific tumor antigen peptide repertoire loaded and/or activated dendritic cell comprising the steps of:
a) exposing in suitable conditions a tumor cell culture expressing said specific tumor antigen peptide repertoire to at least one Pattern Recognition Receptor (PRR) agonist and/or to one inflammatory cytokine to increase the opening of connexin-hemichannels (CxH);
b) culturing dendritic cells with the cell culture supernatant, or a fraction thereof or with purified peptides from the cell culture supernatant, to get specific tumor antigen peptide repertoire loaded and/or activated dendritic cells; and
c) optionally purifying said specific tumor antigen peptide repertoire loaded and/or activated dendritic cells,
wherein step a) and b) are performed simultaneously or in sequence.

3. A method for obtaining an activated tumor antigen-specific CTL comprising the steps of:
a) exposing in suitable conditions a tumor cell culture expressing said specific tumor antigen peptide repertoire to at least one Pattern Recognition Receptor (PRR) agonist and/or to one inflammatory cytokine to increase the opening of connexin-hemichannels (CxH);
b) co-culturing dendritic cells with the cell culture supernatant, or a fraction thereof or with purified peptides from the cell culture supernatant, to get activated tumor antigen-specific CTL,
wherein step a) and b) are performed simultaneously or in sequence.

4. The method according to any one claims 2-3 wherein dendritic cells are autologous or HLA-compatible or -semi-compatible allogenic dendritic cells.

5. The method according to any of previous claims wherein the inflammatory cytokine is gamma-IFN.

6. The method according to any of previous claims, wherein the tumor cell is an established tumor cell line, or a combination of tumor cell lines expressing a specific tumor antigen peptide repertoire or a tumor cell isolated by a tumor affected subject.

7. The method according to any one of the previous claims, wherein the tumor cell derives from solid or non-solid tumors, including melanoma, lung carcinoma, colorectal adenocarcinoma, prostate adenocarcinoma, sarcoma, osteosarcoma, leukemia and T cell-lymphoma and the said specific tumor antigen peptide repertoire is specific for said tumor.

8. The method according to any one of the previous claims, wherein the PRR agonists are Gram-negative, preferably belonging to the *Salmonella* genus, more preferably to non virulent strains of *Salmonella* genus, or Gram-positive bacteria or components thereof,

9. The method according to claim 8 wherein Gram negative bacteria components are LPS and/or flagellin or wherein Gram positive bacteria component is Lipoteichoic acid (LTA).

10. A supernatant or a fraction thereof obtainable by the method according to any one of claims 1 or 5-9.

11. The supernatant of claim 10 or a fraction thereof used in combination with a therapeutic agent, preferably at least one antibody and/or chemotherapeutic and/or immune checkpoint inhibitor.

12. The supernatant according to any one of claims 10-11, or a fraction thereof or the peptides purified by said supernatant, for use as a medicament, preferably for use in the prevention and/or treatment of tumors, more preferably as a tumor immunotherapeutic agent or as tumor vaccine.

13. A specific tumor antigen peptide repertoire loaded and/or activated dendritic cells obtainable by the method according to any one of claims 2 or 4-9, preferably used in combination with a therapeutic agent, preferably at least one antibody and/or chemotherapeutic and/or immune checkpoint inhibitor.

14. The specific tumor antigen peptide repertoire loaded and/or activated dendritic cells according to claim 13, for use as a medicament, preferably for use in the prevention and/or treatment of tumors, more preferably as a tumor immunotherapeutic agent or as tumor vaccine

15. An immunogenic composition or vaccine comprising the supernatant according to any one of claims 10-11 or a fraction thereof, or the peptides purified by said supernatant and/or at least one specific tumor antigen peptide repertoire loaded and/or activated dendritic cell of claim 13 and at least one pharmaceutically acceptable carrier and/or adjuvant.
